(19) Europäisches Patentamt
European Patent Office
Office européen des brevets

(11) **EP 2 060 233 B1**

(12) **EUROPEAN PATENT SPECIFICATION**

(45) Date of publication and mention
of the grant of the patent:
**15.03.2017 Bulletin 2017/11**

(51) Int Cl.:
***A61B 8/08*** *(2006.01)*

(21) Application number: **07806472.2**

(86) International application number:
**PCT/JP2007/066999**

(22) Date of filing: **31.08.2007**

(87) International publication number:
**WO 2008/029728 (13.03.2008 Gazette 2008/11)**

(54) **ULTRASONOGRAPH**

ULTRASCHALLGERÄT

ÉCHOGRAPHE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **01.09.2006 JP 2006237518**

(43) Date of publication of application:
**20.05.2009 Bulletin 2009/21**

(73) Proprietor: **Hitachi, Ltd.
Chiyoda-ku,
Tokyo 100-8280 (JP)**

(72) Inventors:
• **MATSUMURA, Takeshi
Tokyo 101-0021 (JP)**
• **SHIINA, Tsuyoshi
Kyoto-shi, Kyoto 600-8435 (JP)**

• **YAMAKAWA, Makoto
Kyoto-shi, Kyoto 607-8261 (JP)**

(74) Representative: **MERH-IP Matias Erny Reichl
Hoffmann
Patentanwälte PartG mbB
Paul-Heyse-Strasse 29
80336 München (DE)**

(56) References cited:
**EP-A1- 1 629 777        WO-A1-2006/041050
JP-A- 2003 210 460**

• **SATO T. ET AL.: 'Elastography ni Okeru Fuyo
Hizumi Fukin'itsusei no Eikyo no Kansuru
Kosatsu' DAI 18 KAI JAPANESE SOCIETY FOR
MEDICAL AND BIOLOGICAL ENGINEERING
SHUKI TAIKAI RONBUNSHU 05 November 2004,
page 247, XP003021604**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

The present invention relates to an ultrasonic diagnostic apparatus, particularly to an ultrasonic diagnostic apparatus suitable for constructing and displaying a strain image by measuring the strain distribution while pressing biological tissues.

Background Art

As means for diagnosing diseased area by softness or hardness of biological tissues, elastic images are constructed and displayed by pressing the biological tissues using a device such as an ultrasonic probe and calculating strain information of the biological tissues such as distortion or elasticity modulus based on the displacement of the biological tissues caused by the applied pressure.

While an accurate diagnosis of biological tissues can be performed by elasticity modulus which is a quantitative strain information, since elasticity modulus is a value wherein the stress added to each region of the biological tissues is divided by the strain, it is necessary to acquire the stress added to each region of the biological tissues. Acquisition of stress being added to each region is generally carried out by measuring the pressure being added to the skin surface of an object to be examined by a device such as a pressure sensor using press means such as an ultrasonic probe and estimating the stress acted on the biological tissues inside of the object due to the pressure. However, because of the enormous quantity of computation required for presuming the distribution of stress acting on the biological tissues, real time processing of stress acquisition is considered difficult at the present stage. Apparatus configuration is also considered difficult because of the enormous quantity of memory required to perform stress distribution analysis using a method such as the finite element method.

Since it is not yet practical to acquire quantitative elasticity modulus in real time as stated above, elasticity diagnosis is performed mainly by real time strain images based on the strain acquired by differentiating the displacement. In the strain images constructed based on strain information, it is possible to recognize relative difference in strain size as the difference of hardness in biological tissues, thus considered useful for diagnosis since the relative difference of hardness can be acquired though information on quantitative hardness cannot be acquired. They are applied in the regions such as mammary gland tissues, prostatic glandular tissues, and thyroid tissues. The above-mentioned technique for constructing strain images based on the strain information is disclosed in non-patent document 1 and patent documents 1 and 2.

EP 1629 777 A1 describes an ultrasound probe including an ultrasound wave transmit/receive surface coming into contact with a contact surface of a subject, and a pressing mechanism for performing a pressing operation for applying a pressure to the contact surface perpendicularly to the ultrasound wave transmit/receive surface via the ultrasound wave transmit/receive surface.

Non-patent document 1: Karsten Mark Hiltawsky, et al. , Freehand ultrasound elastography of breast lesions: Clinical results. Ultrasound in Med. & Biol. , Vol. 27, No. 11, pp.1461-1469, 2001.

Patent Document 1: JP-P2004-229459
Patent Document 2: WO2006/041050-A1

Disclosure of the Invention

Problems to be Solved

The prior art disclosed in the above-mentioned documents acquires the displacement of each region of biological tissues which varies in compliance with pressure on the basis of a pair of frame data acquired at different times, and obtains strain distribution of the biological tissues from acquired displacement of each region. However, the fact that the stress acting on biological tissues gets attenuated as the depth of the region from pressing means gets deeper is not taken into consideration. Therefore, there are cases that the tissues having the same elasticity in the depth direction are measured as having different values depending on the depth from the pressing means, which could lead to an inaccurate diagnosis.

The pressure added to the object using pressing means such as an ultrasonic probe is transmitted by elastic waves from the contact surface between the pressing means and the object in the depth direction of the object. In the transmission process, the elastic waves are transmitted to a wide range while being diffracted, thus the stress per unit area are attenuated depending on the depth. As a result, the stress is attenuated as it reaches the deeper region, and the displacement gets smaller in accordance with the attenuation. For example, when there are tissues having the same hardness in the shallow part and the deep part from the vicinity of the pressing means, since the strain in the deep part is measured smaller than the strain in the shallow part, there is a potential of misdiagnosing the tissues in the deep part as hard tissues since the tissue in the deep part has smaller displacement than the shallow part.

The objective of the present invention is to obtain appropriate strain information regardless of the depth from the pressing means.

The object is solved by the invention according to the independent claim. Further preferred developments are described by the dependent claims.

Means for Solving the Problem

The ultrasonic diagnostic apparatus comprises:

an ultrasonic probe for transmitting/receiving ultrasonic waves to/from an object to be examined;
pressing means for pressing biological tissues of the object;
transmission means for transmitting ultrasonic waves to the biological tissues by the ultrasonic probe;
reception means for receiving the reflected echo signals generated from the object by the ultrasonic probe;
strain information calculating means for obtaining strain distribution of biological tissues based on a pair of frame data acquired at different times that are received by the reception means;
strain image constructing means for constructing strain images based on the strain distribution obtained by the strain information calculating means; and
display means for displaying the strain images,
characterized in further comprising:

strain distribution correcting means for correcting the strain distribution using a strain distribution correcting function being set depending on the pressure condition applied by the pressing means.

It further comprises storage means for obtaining and storing the strain distribution correcting function depending on the press condition by the pressing means, wherein the strain distribution correcting means corrects the strain distribution by the stored strain distribution correcting function.

It also comprises storage means for obtaining and storing the strain distribution correcting function for each coordinate position of the strain distribution, wherein the strain distribution correcting means corrects the strain distribution by the stored strain distribution correcting function.

Also, in place of the storage means, it comprises displacement calculation means for correcting the displacement distribution of biological tissues obtained on the basis of a pair of frame data by the displacement distribution correcting function being set depending on the pressure condition applied by the pressing means, wherein the strain calculation means obtains the strain distribution based on the corrected displacement distribution.

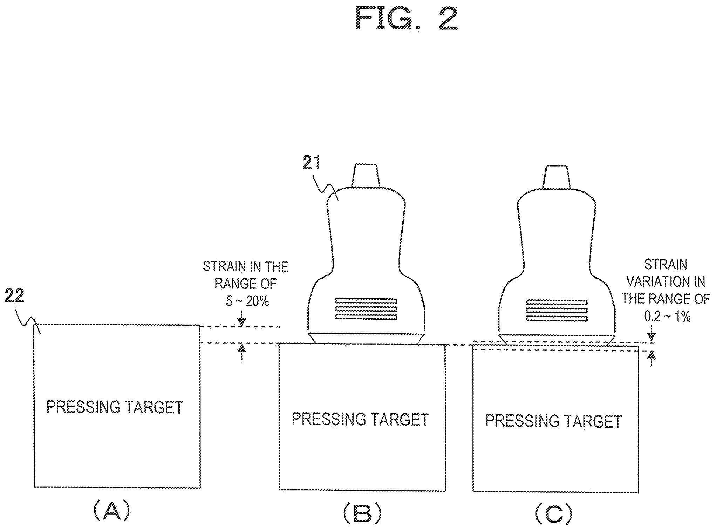

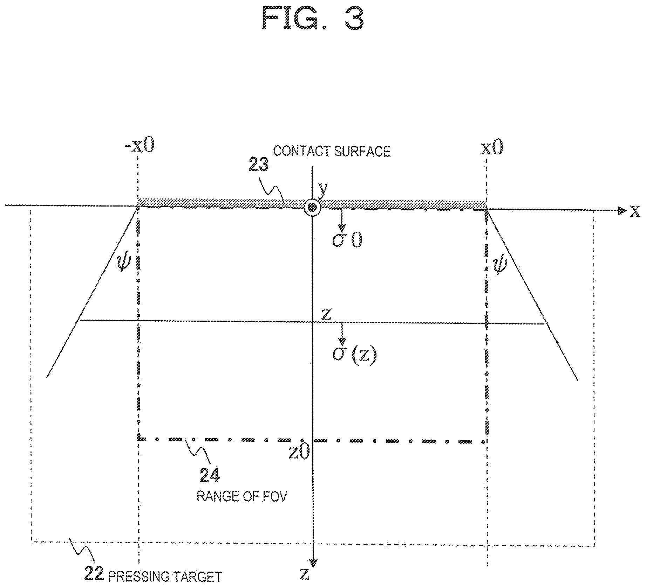

Here, the principle of the present disclosure will be described referring to Fig. 2 and Fig. 3. For example, as shown in Fig. 2, an example that a linear ultrasonic probe 21 is used as pressing means and a phantom having uniform hardness is used as a pressure target 22 will be described. Generally, as shown in Fig. 2(B), strain measurement is performed by applying the ultrasonic transmission/reception surface of the ultrasonic probe 21 to a pressing target 22 shown in Fig. 2(A), and from the condition thereof by adjusting the pressure so as to generate compression (strain change) in the range of 5 ∼ 20% as shown in Fig. 2(C). Fig. 3 is for explaining stress distribution in a section parallel to an x-axis (tomographic section) by representing a contact surface 23 between the ultrasonic probe 21 and the pressing target 22 by an x-y axis and depth direction by a z-axis.

The contact surface 23 should have sufficient hardness in comparison to the pressing target 22 so its shape does not change by the pressure within the measurement range. Also, the length of the contact surface 23 in the x-axis direction is set as $2 \cdot x0$, and the length in the y-axis direction is set as $2 \cdot y0$. A stress $\sigma$ in the contact surface 23 is set as $\sigma = \sigma 0$ (z=0). It is assumed now that elastic waves of the pressure added to the contact surface 23 is spread and transmitted at a diffraction angle $\psi$ with respect to the press direction, and that a stress $\sigma(z)$ in the z-direction on an arbitrary "xy" surface (z=constant) in the channel region of the elastic waves is a steady value without depending on the coordinate of "x,y". In other words, it is assumed that "external force added by pressure to the pressing target = stress × area" is constant regardless of depth.

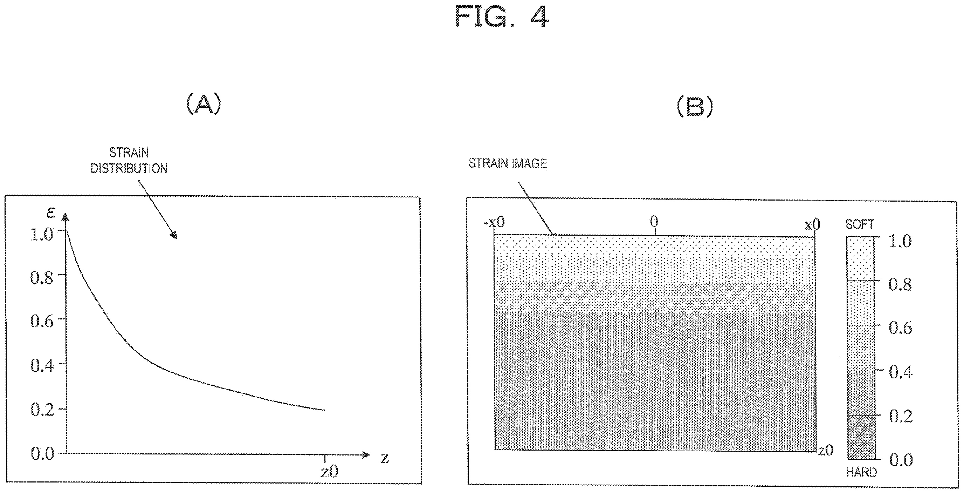

When the strain is measured with respect to a constant FOV range 24 of the pressing target 22 in the condition of the above-described assumption, distribution of the strain $\varepsilon$ in the central axis of the FOV range 24 is such that the strain $\varepsilon$ decreases as the depth gets deeper as shown in Fig. 4(A). In other words, since the pressure added by the ultrasonic probe 21 spreads and transmits within the pressing target 22, the stress acting on the biological tissues are attenuated in compliance with the depth and the strain of the tissues in a deeper part in the FOV range 24 is measured smaller than the strain of the tissues in a shallow part. While attenuation of stress that acts on biological tissues occurs due to the factors other than diffraction transmission of elastic waves, the attenuation depends on pressure measurement condition such as the shape of the contact surface between pressing means and an object, size of the pressing target (boundary condition) and a diffraction angle $\psi$. As for the size of the pressing target (boundary condition), when the pressing target is large enough in comparison to the contact surface, the stress attenuates according to a predetermined function as to be described below in embodiment 1. However, for example, when the width of the pressing target is small, that is the width of the contact surface is narrower than the width of the ultrasonic transmission/reception surface, the stress is attenuated in the vicinity of the contact surface which makes it harder to reach the deep part, since both sides of the pressing target can change their shape without restriction. Therefore, since the manner of stress attenuation differs depending on the boundary condition such as the size or shape of the pressing target, the boundary condition should be taken into consideration as measurement condition.

Given this factor, the present disclosure measures the strain distribution for each pressure measuring condition in advance, and sets a strain distribution correcting function to calculate the strain distribution in the case that the stress in the contact surface does not get attenuated even in an arbitrary depth. Then it is set so that the strain distribution is corrected by the distribution correcting function so as to obtain appropriate strain information regardless of depth or direction from the pressing means.

Brief Description of the Diagrams

Fig. 1 is a block configuration diagram of the entire ultrasonic diagnostic apparatus in the embodiment 1 related to the present disclosure.
Fig. 2 illustrates the operation for pressing a pressing target using a linear ultrasonic probe.
Fig. 3 illustrates stress distribution in the depth direction of the pressing target in the embodiment 1 being pressed by a linear ultrasonic probe.
Fig. 4 illustrates the fact that the strain distribution attenuates by the stress attenuation in the embodiment 1 being pressed by a linear ultrasonic probe.
Fig. 5 illustrates that strain distribution can be corrected properly using a strain distribution correcting function in the embodiment 1.
Fig. 6 shows a configuration diagram of embodiment 2 of pressing means wherein a circular balloon is to be attached to a transrectal ultrasonic probe.
Fig. 7 illustrates press direction generated by a balloon in the embodiment 2.
Fig. 8 illustrates attenuation of stress in the FOV range in the embodiment 2.
Fig. 9 shows a configuration in embodiment 3 of pressing means wherein a tubelike balloon is attached to a transrectal ultrasonic probe.
Fig. 10 illustrates attenuation of stress in the FOV range in embodiment 4 pressed by a transrectal ultrasonic probe.
Fig. 11 illustrates embodiment 6 which makes a strain distribution correcting function to be arbitrarily fine-adjustable in the depth direction.

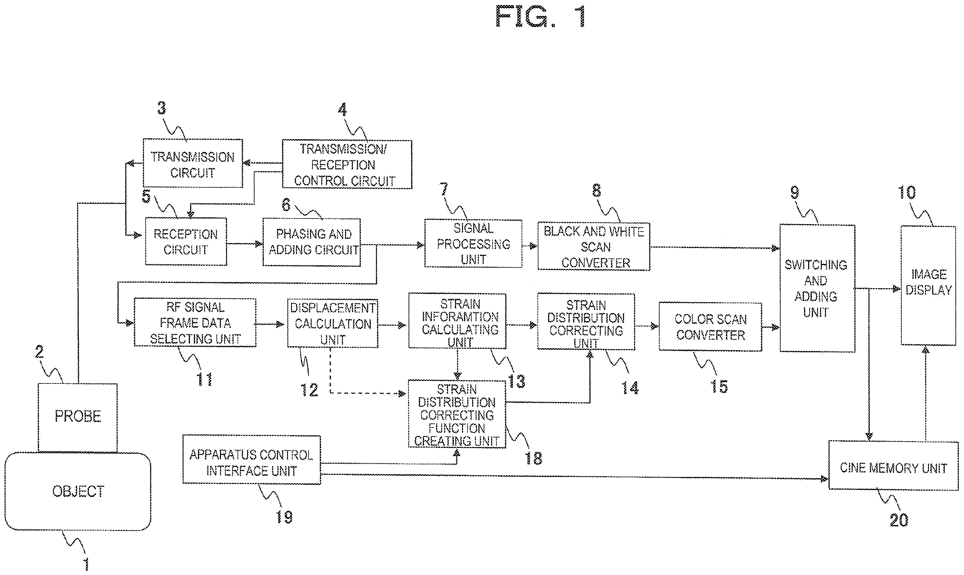

Hereinafter, the present disclosure will be described based on embodiments. Fig. 1 shows a block configuration diagram of an entire ultrasonic diagnostic apparatus in the first embodiment related to the present disclosure. As shown in the diagram, an ultrasonic probe (hereinafter abbreviated as a probe) 2 to be applied to an object 1 is formed having a plurality of transducers that transmit/receive ultrasonic waves to/from an object 1. The probe 2 is driven by the ultrasonic pulses provided from a transmission circuit 3. A transmission/reception control circuit 4 is for controlling transmission timing of the ultrasonic pulses for driving the plurality of transducers of the probe 2, and forming ultrasonic beams toward a focal point set in the object 1. Also, it electronically scans ultrasonic beams in the array direction of the transducers of the probe 2.

On the other hand, the probe 2 receives the reflected echo signals generated from the object 1 and outputs them to a reception circuit 5. The reception circuit 5 receives the reflected echo signals in accordance with the timing signals inputted from the transmission/reception control circuit 4 and performs reception process such as amplification. The reflected echo signals processed by the reception circuit 5 are amplified by combining and adding phases of the reflected echo signals received by the plurality of transducers in a phasing and adding circuit 4. The reflected echo signals processed by the reception circuit 5 are amplified by adjusting and adding phases of the reflected echo signals received by the plurality of transducers in the phasing and adding circuit 6. The reflected echo signals phased and added in the phasing and adding circuit 6 are inputted to the signal processing unit 7, and receive signal processing such as gain compensation, log compression, detection, edge enhancement and filtering.

The reflected echo signals processed by the signal processing unit 7 are transmitted to a black and white scan converter 8, and converted into 2-dimensional tomographic data (digital data) corresponding to the scan plane of the ultrasonic beams. Image reconstruction means of tomographic images (B-mode images) is configured by the above-described signal processing unit 7 and the black and white scan converter 8. The tomographic image data outputted from the black and white scan converter 8 are provided to an image display 10 via the switching and adding circuit 9, and the B-mode images are displayed.

On the other hand, the reflected echo signals outputted from the phasing and adding circuit 6 are transmitted to a RF signal frame data selecting unit 11. The RF signal frame data selecting unit 11 selects a reflected echo signal group corresponding to the scan plane (tomographic plane) of the ultrasonic beams as frame data, obtains a plurality of frames of data, and stores them in a device such as a memory. A displacement calculation unit 12 sequentially receives the plurality of frame data acquired at different times stored in the RF signal frame data selecting unit 11, obtains displacement vector of the plurality of measuring points on a tomographic plane based on the received pair of frame data and outputs them as displacement frame data to a strain information calculating unit 13.

The strain information calculating unit 13 of the present embodiment is configured so as to obtain strain of the biological tissues in the respective measuring points based on displacement frame data. The strain distribution (frame data) obtained in the strain information calculating unit 13 is to be outputted to a strain distribution correcting unit 14.

The strain distribution correcting unit 14 corrects the strain distribution inputted from the strain information calculating unit 13 by the strain distribution correcting function outputted from a strain distribution correcting function creating unit 18. Then it performs a variety of imaging process such as a smoothing process in the coordinate plane, contrast optimization process and a smoothing process between the frames in the time axis direction with respect to the strain information by the corrected strain distribution, and outputs them to a color scan converter 15.

The color scan converter 15 receives the strain distribution corrected by the strain distribution correcting unit 14 and constructs color strain images by appending a hue code for each pixel of the frame data of strain distribution in accordance with the set strain color map.

The color strain images constructed by the color scan converter 15 are displayed on the image display 10 via the switching and adding unit 9. The switching and adding unit 9 is configured having a function for inputting black and white tomographic images outputted from the black and white scan converter 8 and color strain images outputted from the color scan converter 15, and displays one of them by switching both images, a function for making one of the images transparent, performing additive synthesis and displaying by superimposing over the image display 10, and a function for juxtaposing and displaying both images. Also, the image data outputted from the switching and adding unit 9 is to be stored in a cine memory 20 under the control of an apparatus control interface unit 19. The image data stored in the cine memory 20 are to be displayed on the image display 10 under the control of the apparatus control interface unit 19.

The strain distribution correcting function creating unit 18 related to the feature of the present embodiment reads a pressure measurement condition inputted from the apparatus control interface unit 19 such as the shape of a contact surface between the pressing means (a probe 2 in Fig. 1) and an object 1, the size of the FOV range of a measurement target (boundary condition) or a diffraction angle $\psi$. Then the strain distribution correcting function creating unit 18 calculates or selects and sets the strain distribution correcting function described in embodiments below. The set strain distribution correcting function is outputted to the strain distribution correcting unit 14.

Basic operation of such configured present embodiment will be described. First, ultrasonic beams are scanned to the object 1 by adding pressure to the object 1 by the probe 2, and the probe 2 continually receives the reflected echo signals from the scan plane. Then a topographic image is reconstructed by the signal processing unit 7 or the black and white scan converter 8 based on the reflected echo signals outputted from the phasing and adding circuit 6, and the reconstructed image is displayed on the image display 10 by the switching and adding device 9.

On the other hand, the RF signal frame data selecting unit 11 reads the reflected echo signals, repeatedly obtains frame data by synchronizing the signals to the frame rate and stores the obtained data to a built-in frame memory in chronological order. Then by setting a pair of frame data acquired at different times as a unit, it continually selects plural pairs of frame data and outputs them to a displacement calculation unit 12. The displacement calculation unit 12 performs one-dimensional or two-dimensional correlation processing on a pair of selected frame data, measures the displacement in the plurality of measuring points on a scan plane, and generates displacement frame data. The block matching method or the gradient method disclosed in documents such as JP-A-H5-317313 are commonly known as the detection method of displacement vectors. The block matching method divides an image into blocks formed by, for example, N $\times$ N pixels, searches from the previous frame for the most approximated block to the target block of the present frame, and obtains the displacement of the measuring point based on the searched block. Also, displacement can be obtained by calculating auto-correlation in the same region of a pair of RF signal frame data.

The strain information calculating unit 13 obtains the strain variation of the respective measuring points by reading frame data of the strain, and outputs the strain distribution (frame data) to the strain distribution correcting unit 14. Calculation of displacement variation can be carried out, as commonly known, by performing spatial differentiation on the displacement of the respective measuring points and calculating strain variation $\Delta\varepsilon$ of the respective measuring points. Also, as proposed in Non-patent document 1, by setting a region of interest "ROI" and the reference region ROI0 in a FOV range and obtaining the average value of the strain variation $\Delta\varepsilon$ and $\Delta\varepsilon$ 0 in those regions, differentiation of benignancy/malignancy of tissues can be performed by the ratio of the obtained average values (average of $\Delta\varepsilon$ 0/average value of $\Delta\varepsilon$).

The strain distribution correcting unit 14 performs processing such as a smoothing process on the inputted strain distribution, corrects the strain distribution using a strain distribution correcting function inputted from the strain distribution correcting function creating unit 18, and outputs the strain information based on the corrected strain distribution to the color scan converter 15. The color scan converter 15 generates color strain images based on the strain distribution. A color strain image is colored for each pixel unit in accordance with the strain of frame data by, for example, 256 shades of hue gradation. In place of the color scan converter 15, a black and white scan converter may be used. In this case, benignancy or malignancy of tissues can be differentiated by the method such as making luminance to be bright for the region having large strain and making luminance to be dark for the region having small strain.

Hereinafter, concrete embodiments of the strain distribution correction based on the difference of pressing means and the difference of pressure measurement condition will be described using the present embodiment. Each embodiment will be performed by the devices which are the feature of the present embodiment such as the strain information calculating unit 13, strain distribution correcting unit 14, strain distribution correcting function creating unit 18 and apparatus control interface unit 19.

In the present disclosure, strain distribution of Fig. 4(A) is measured in advance for each pressure measurement condition, and a strain distribution correcting function wherein the stress on the contact surface does not get attenuated even in an arbitrary depth is set in the strain distribution correcting function creating unit 18. Then the strain distribution correcting unit 14 can obtain adequate strain information regardless of depth or direction from the pressing means by correcting the strain distribution obtained from the strain information calculating unit 13 using a strain distribution correcting function.

Embodiment 1

In the present embodiment 1, correction is performed on strain information of the case using a linear-type probe 21 shown in Fig. 2 as pressing means and that the ultrasonic transmission/reception surface (contact surface) of the probe 21 is pushed and pressed against the object. The contact surface of the linear-type probe 21 has sufficient hardness compared to the object 1, and does not change its shape by the pressure within the measurement range.

Also, as shown in Fig. 3, the length of a contact surface 23 in the x-axis direction is set as $2 \cdot x0$, the length in the y-axis direction is set as $2 \cdot y0$. and the stress $\sigma$ on the contact surface 23 is set as $\sigma=\sigma0(z=0)$. It is now assumed that the elastic waves of the pressure added to the contact surface 23 is spread and transmitted at diffraction angle $\psi$ with respect to the pressure direction, and in an arbitrary "xy" plane (z=constant) in the channel region of the elastic waves, the model that the stress $\sigma(z)$ in the z-direction is a steady value without depending on the coordinate of "x,y" is set, as shown in the following formula (1). In other words, pressure (external force) added to the object 1 = stressXarea, is constant regardless of the depth. In the formula (1), "ds" is a minute area element.

$$\int (z)\ ds\ =\ constant \qquad (1)$$

Also, a spreading range Ux(z) of the depth "z" in the x-direction with a diffraction angle $\psi$ can be expressed by the following formula (2).

$$Ux(z)\ =\ 2(x0\ +\ z\cdot\tan\psi) \qquad (2)$$

In the same manner, the spreading range Uy(z) of the depth "z" in the y-direction with a diffraction angle $\psi$ can be expressed by the following formula (3).

$$Uy(z)\ =\ 2(y0\ +\ z\cdot\tan\psi) \qquad (3)$$

From these formulas, the following formula (4) can be expressed.

$$\sigma 0 \cdot (2x0) \cdot (2y0) \fallingdotseq \sigma(z) \cdot 2(x0+z \cdot \tan\psi) \cdot 2(y0+z \cdot \tan\psi) \quad (4)$$

From the formula (4), the stress in an arbitrary position of the z-axis can be expressed by the following formula (5).

$$\sigma(z) = \sigma 0 \cdot (x0 \cdot y0)/\{(x0+z \cdot \tan\psi) \cdot (y0+z \cdot \tan\psi)\} \quad (5)$$

Here, while the diffraction angle $\psi$ depends on the frequency of the elastic waves (frequency of the repeated pressing operation), in the condition that, for example, $\psi = \pi / 4$, the formula (5) is expressed as the formula (6).

$$\sigma(z) = \sigma 0 \cdot \{x0 \cdot y0\}/\{(x0+z) \cdot (y0+z)\} \quad (6)$$

Also, in the case of a shallow region in the vicinity of the contact surface, under the condition that $z << x0, y0$ the stress can be expressed as $\sigma(z) \fallingdotseq \sigma 0$ (constant). In the case of a deep region, the stress can be expressed as $\sigma(z) \fallingdotseq \sigma 0 \cdot \{x0 \cdot y0\}/\{z \cdot z\}$ under the condition that $z >> x0.y0$.

Therefore, in the deep region, the stress gets drastically changed and attenuated in the relationship of $1/z^2$. As a result, even the stress $\sigma(z)$ of the biological tissues having uniform hardness gets attenuated as they are transmitted, and the strain distribution is acquired with the attenuated strain value.

In the present embodiment 1, the strain distribution correcting unit 14 corrects strain distribution considering the above-mentioned stress attenuation, and develops strain information based on the corrected strain distribution (hereinafter, referred to as the corrected strain distribution). The concrete correcting method of the strain distribution in the present embodiment 1 will be described in detail.

It is assumed that the measurement of strain distribution is performed by the probe 21 in Fig. 2 under the above-described pressure measuring condition. It is also assumed that elasticity of the biological tissues in an FOV range 24 is uniform, and the strain distribution data obtained by the measurement is set as $\varepsilon(x,z)$. The FOV range at this time is set as $-x0 \leqq x \leqq x0$, $0 \leqq z \leqq z0$. In this condition, for example, the strain distribution $\varepsilon(0, z)$ in the depth direction on the center line $x=0$ turns out as the distribution being decreased toward the depth as shown in Fig. 4(A) due to attenuation of the stress. Then since the strain information based on the strain distribution $\varepsilon(x,z)$ turns out as shown in Fig. 4(B) and the strain becomes smaller as the region gets deeper, there is a possibility of misidentifying that a hard region exists in the deep region.

Given this factor, in the present embodiment, the following formula (7) is defined in the strain distribution correcting function creating unit 18 by setting the strain distribution correcting function w(z) and considering the above-described formula (6). The strain distribution correcting function w(z) is the inverse number of the strain attenuation amount shown in the formula (6).

$$w(z) = \{(x0+z) \cdot (y0+z)\}/\{x0 \cdot y0\} \quad (7)$$

Further, the strain distribution correcting unit 14 obtains the corrected strain distribution $\varepsilon'(x, z)$ by the following formula (8).

$$\varepsilon'(x,z) = w(z) \cdot \varepsilon(x,z) \quad (8)$$

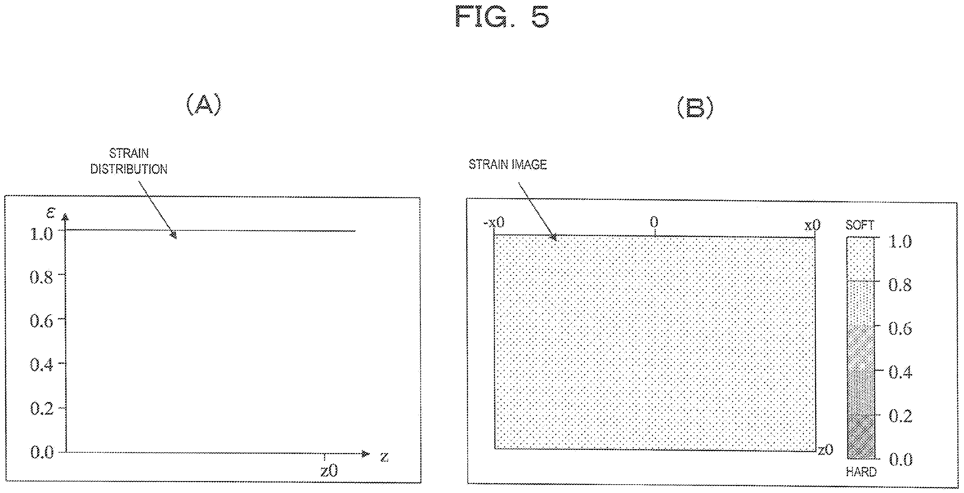

The strain distribution correcting unit 14 corrects strain distribution by multiplying the inverse number of the stress attenuation amount by the strain distribution. In other words , the strain distribution correcting unit 14 corrects strain distribution using the strain distribution correcting function "w(z)" outputted from the strain distribution correcting function creating unit 18 in prospect of the stress attenuation. In this manner, the corrected strain distribution is distributed flatly with respect to the depth direction as shown in Fig. 5(A), and an elastic image by the strain information based on the corrected strain distribution also turns out not having difference in strain size over the entire image as shown in Fig. 5(B), whereby making it possible to avoid misidentification.

Also, in the case of measuring the biological tissues having the regions with different hardness as a measurement target, difference of the hardness can be obtained more accurately by applying the strain distribution correcting function "w(z)".

While the approximation was performed using the formula (6) assuming that the diffraction angle $\psi = \pi/4$, the present invention does not have to be limited thereto, and the angle may be set variably. Also, the strain distribution correcting function w(z) may be set by repeatedly setting the diffraction angle $\psi$ of elastic waves as the function of the pressure operation frequency, repeatedly measuring the pressure operation frequency and assuming the stress attenuation using the formula (5).

Embodiment 2

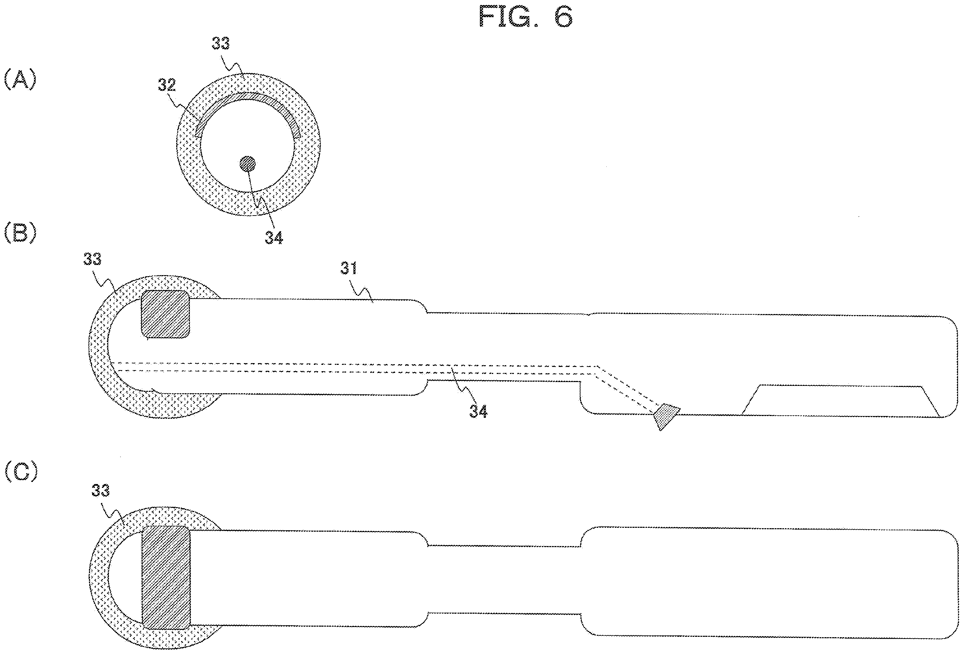

In embodiment 2, correction is made on the strain information of the case using a convex-type transrectal probe 31 shown in Fig. 6, and that an object is pressed by expanding/contracting a spherical-shaped balloon 33 which is attached to the end of the transrectal probe as pressing means. The balloon 33 is an example of being attached encompassing a convex-type ultrasonic transmission/reception surface 32, and is expended/contracted by charging/discharging water from a syringe, etc. via a fluid channel 34 communicated therein.

As previously described, attenuation of stress depends on the shape of a contact surface for adding pressure, and also depends on the transmission of stress being spread by the diffraction of elastic waves. In other words, attenuation of stress appears prominently in pressure measuring condition having a wide FOV range with respect to the contact surface area, to which a probe of intra-luminal type such as the transrectal probe 31 in the embodiment 2 is relevant. A transvaginal probe and transesophgeal probe, etc. can be cited as the other body-inserting probes.

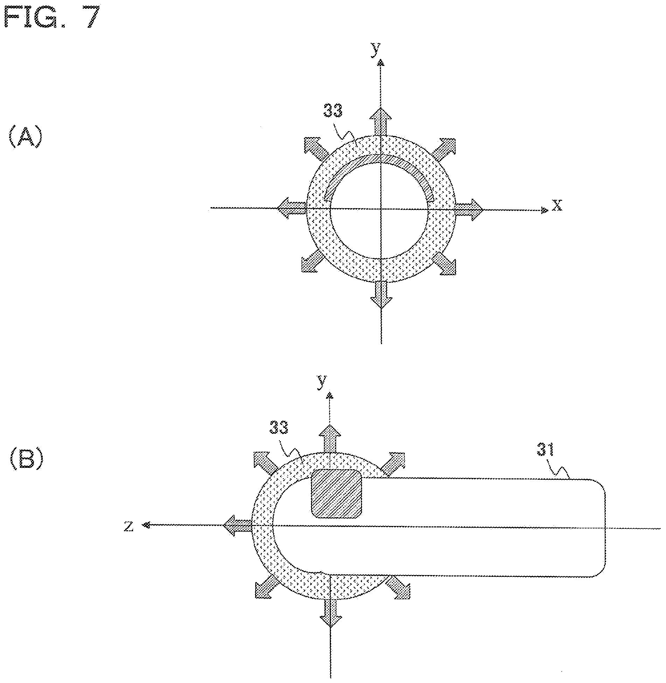

The method for measuring elasticity by adding pressure using a spherical-shaped balloon 33 as shown in Fig. 6 is proposed in Patent Document 1. In the case of the embodiment 2, when the film surface of the balloon 33 contacts the skin surface in a body cavity of the object and liquid is charged into the balloon 33, the direction that the film surface extends to press biological tissues is the normal line direction of the spherical surface as shown in Fig. 7. Regarding the "xy" plane shown in Fig. 7(A), transmission of stress will be described under the same condition as the embodiment 1 and the pressure can be applied to a pressure target with sufficient force while maintaining the spherical surface.

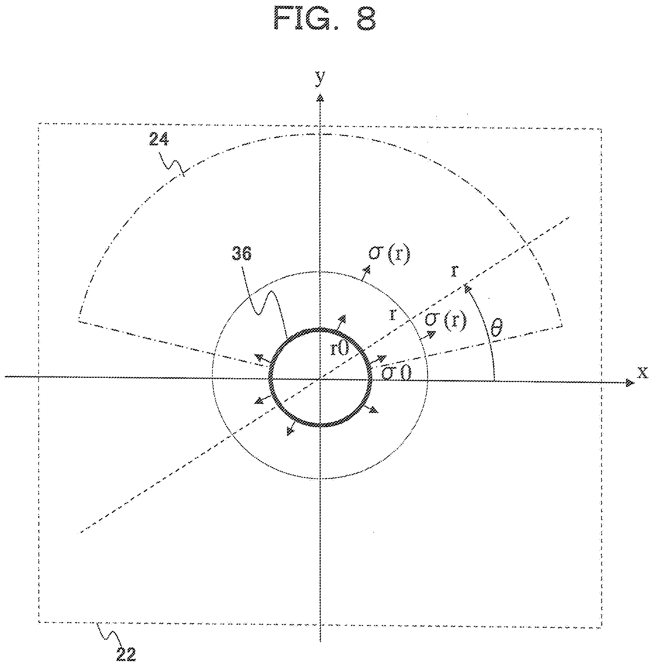

In the same manner as the pressure operation in Fig. 2, after pressing in the initial state, the biological tissues are pressed by repeatedly adding and reducing the pressure force. Now, the curvature radius of the balloon 33 in the initial state is set as "r0", and the stress on the contact surface $\sigma$ is set as $\sigma = \sigma0(r=r0)$. Then, as shown in Fig. 8, the coordinate of the measuring point on the "xy" plane is specified as $(r, \theta)$. Also, the elastic waves generated on the contact surface 36 are assumed to be transmitted toward normal line direction of the spherical surface as spherical waves. At this time, the model that the "force = stress X area" is constant without depending on the depth is developed as in the embodiment 1. In other words, in accordance with the formula (1), the following formula (9) can be expressed.

$$\sigma0 \cdot 4\pi(r0)^2 = \sigma(r) \cdot 4\pi(r)^2 \qquad (9)$$

Therefore, $\sigma(r)$ can be obtained by the following formula (10).

$$\sigma(r) = \sigma0 \cdot (r0/r)^2 \qquad (10)$$

As is apparent from the formula (10), $\sigma(r)$ is drastically changed and attenuated in the relationship of $1/r^2$. Given this factor, in the present embodiment, the strain distribution correcting function creating unit 18 defines the following formula (11) as the strain distribution correcting function w(r) by coupling with the formula (10). The strain distribution correcting function w(r) is the inverse number of the stress attenuation amount shown in the formula (10).

$$w(r) = (r/r0)^2 \qquad (11)$$

The strain distribution correcting unit 14 corrects strain distribution by the strain distribution correcting function w(r), and obtains the strain distribution $\varepsilon'(r, \theta)$ by the following formula (12).

$$\varepsilon'(r,\theta) = w(r) \times \varepsilon(r,\theta) \qquad (12)$$

In accordance with the present embodiment, the strain distribution correcting unit 14 corrects strain distribution by multiplying the inverse number of the stress attenuation amount by the strain distribution as the embodiment 1. Difference in stress size due to attenuation of stress can be eliminated in the entire region of the corrected strain information, and misidentification in diagnosis due to strain information based on the corrected strain distribution can be prevented. Also, in the case of measuring the biological tissues having the regions with different hardness as a measurement target, the difference of hardness can be accurately acquired by applying the strain distribution correcting function w(r).

Embodiment 3

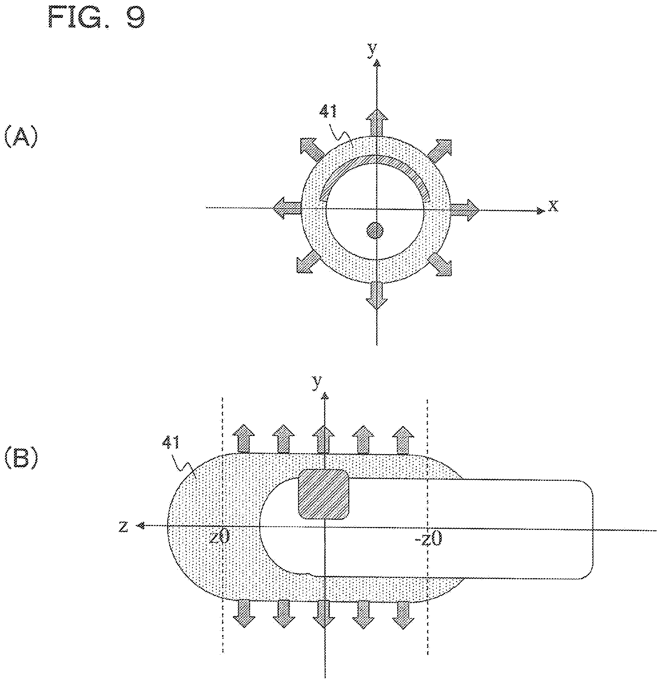

In the embodiment 2, the case of using a balloon 33 having a spherical-shaped membrane as pressing means is described. In the present embodiment 3, as shown in Fig. 9(A) and (B), an example of a strain distribution correcting function in the case of using a balloon 41 having a cylindrical-shaped membrane as pressing means will be described. The balloon 41 in the present embodiment contacts a pressing target by its cylindrical-shaped film surface, expands/contracts while maintaining the cylindrical film surface, and applies pressure in the normal line directions of the cylindrical film surface. In the case of the present embodiment that the contact surface between the balloon 41 and the pressing target is very wide and the length 2·z0 in the z-axis direction of Fig. 9(B) is sufficiently large compared to the size of radius "r" of the FOV range, attenuation can be ignored regarding stress transmission within the "yz" plane in the same manner as the shallow part in the embodiment 1. In other words, when pressure is applied using a sufficiently large cylindrical-shaped balloon 41, stress attenuation needs to be considered only within the "xy" plane indicated in Fig. 8. The following formulas (13) and (14) are set from the model condition of the formula (1).

$$\sigma 0 \cdot 2\pi r0 = \sigma(r) \cdot 2\pi r \qquad (13)$$

$$\sigma(r) = \sigma 0 \cdot (r0/r) \qquad (14)$$

By these formulas, it is apparent that the stress in the present embodiment is drastically attenuated in the relationship of 1/r. Given this factor, based on the formula (14), the strain distribution correcting function creating unit 18 defines the following formula (15) as the strain distribution correcting function w(r). The strain distribution correcting function w(r) is the inverse number of the stress attenuation amount indicated in the formula (14).

$$w(r) = (r/r0) \qquad (15)$$

Then the strain distribution correcting unit 14 corrects the measured strain distribution $\varepsilon(r,\theta)$ by the strain distribution correcting function w(r), and obtains the corrected strain distribution $\varepsilon'(r, \theta)$ by the following formula (16).

$$\varepsilon'(r,\theta) = w(r) \times \varepsilon(r,\theta) \qquad (16)$$

By this formula, in accordance with the present embodiment, the strain distribution correcting unit 14 corrects the strain distribution by multiplying the inverse number of the stress attenuation amount by the stress distribution in the same manner as the embodiments 1 and 2. Thus the misidentification caused by the strain information based on the corrected strain distribution can be prevented. Also, in the case of measuring the biological tissues having regions with different hardness, the difference of hardness can be accurately acquired by applying the strain distribution correcting function w(r).

Also, as is evidenced that the stress is attenuated in accordance with $1/r^2$ in the case of the balloon in embodiment 2 and the stress is attenuated in accordance with 1/r in the case of the balloon in the embodiment 3, attenuation characteristic of stress varies when a balloon is used as pressing means depending on the form of expansion/contraction of the balloon and the size of the contact surface.

Also, the strain correcting method of the present embodiment 3 can be applied in the case of measuring the strain by using the pressure force added to biological tissues of a blood vessel wall or its surrounding tissues utilizing the phenomenon of expansion/contraction caused by the motion of a blood vessel wall as pressing means. For example, it can be applied to diagnoses such as thyroid diagnosis using pulses of carotid artery or diagnosis of deep venous thrombosis using arterial pulses of a lower limb.

Furthermore, in place of the method using pulses, it can also be applied to the case of measuring strain using pressure force added to biological tissues of a blood vessel wall or its surrounding tissues by utilizing expansion/contraction of a balloon inserted into the blood vessel as pressing means such as a balloon catheter.

Embodiment 4

The embodiment 4 is an example of correcting strain distribution in the case of using a convex-type probe 2 itself as pressing means. In the embodiments 1 ∼ 3, stress force which is uniform in the depth direction of a pressing target within the FOV range was the press measurement condition for stress attenuation. The present embodiment 4 is an example of strain correction in the case of adding pressure force to a pressing target using a transrectal probe 31 shown in Fig. 6 as pressing means without using a balloon.

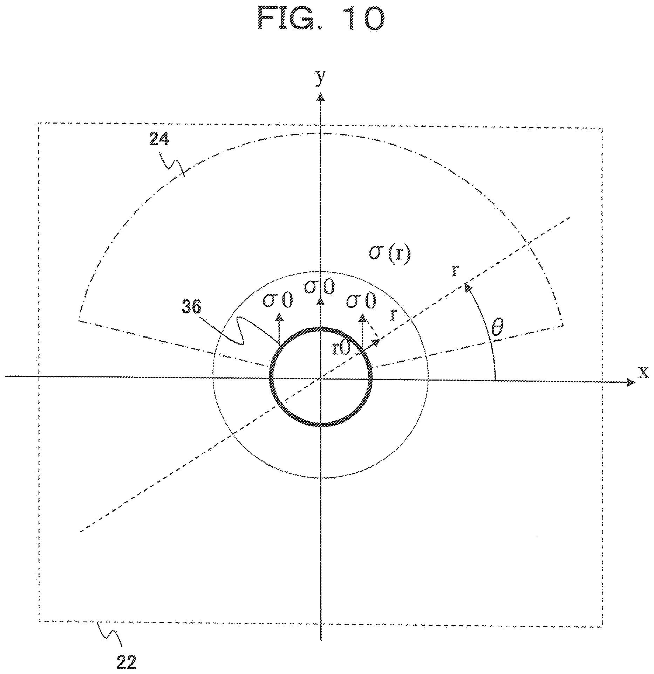

The convex-type probe 2 has curvature in the long-axis direction of the ultrasonic transmission/reception surface 32, and presses the pressing target, for example, while moving the center of the long axis toward normal line directions as shown in Fig. 10. In this case, pressure direction on the contact surface is different from the depth direction of the fan-shaped FOV range, component force of the depth direction in the FOV range of the pressure force in the y-axis direction added to the contact surface becomes effective pressure force toward the ultrasonic beam direction. Therefore, the pressure measuring condition of the embodiment 4 brings out the characteristic that the pressure in the contact surface becomes inhomogeneous in accordance with the direction of ultrasonic beams. As a result, stress distribution becomes inhomogeneous in the FOV range which makes strain distribution also inhomogeneous, which could lead to a misdiagnosis.

In Fig. 10, when it is assumed that the probe 2 is moved to the y-direction in the diagram to apply pressure, the range of pressure direction is at least $0 < \theta < \pi$, and the range of the direction without pressure is $\pi \leqq \theta \leqq 2\pi$. In the range of pressure direction, steady size pressure σ0 is added in the y-axis direction as shown in the diagram in any coordinate (r0, $\theta$) of the contact surface. Unlike the case of a balloon, component of pressure in the normal line direction on the contact surface varies depending on the coordinate (r0, $\theta$). Given this factor, the component σ0' in the normal line direction varies by "sin $\theta$" as shown in the following formula (17). The $\theta$ is an angle formed by the normal line and the x-axis.

$$\sigma 0'(\theta) = \sigma 0 \cdot \sin\theta \qquad (17)$$

In accordance with this formula, in the case of performing diagnosis in a wide FOV range, stress and strain becomes large in the central part of FOV range ($\theta$ = in the vicinity of $\pi/2$), and stress becomes small in both sides of the FOV range ($\theta$ = 0 or the vicinity of $\pi$). Consequently, measured value of the strain within the FOV range also varies depending on the stress , which could lead to misdiagnosing that, for example, there are harder tissues in the side parts than in the central part. Given this factor, in the present embodiment considering nonuniformity of pressure measuring condition, the strain distribution correcting function creating unit 18 sets a strain distribution correcting function w($\theta$) as below, and the strain distribution correcting unit 14 corrects the strain distribution.

First, the strain distribution correcting function creating unit 18 sets the strain distribution correcting function w($\theta$) as the following formula (18) based on the formula (17). The strain distribution correcting function w($\theta$) is the inverse number of the stress attenuation amount indicated in the formula (17).

$$w(\theta) = 1/(\sin\theta) \qquad (18)$$

Therefore, the strain distribution correcting unit 14 obtains the corrected strain distribution ε'(r, $\theta$) by correcting the measured strain distribution ε(r, $\theta$) by the following formula (19).

$$\varepsilon'(r,\theta) = w(\theta) \times \varepsilon(r,\theta) \qquad (19)$$

The strain distribution correcting unit 14 corrects the strain distribution by multiplying the inverse number of stress attenuation amount by the strain distribution. Further, in accordance with the stress that attenuates in compliance with the depth, it can be corrected in the same manner using, for example, the strain distribution correcting function w(r) in the embodiment 3. In other words, depending on size of FOV range, particularly the depth range, effect of stress distribution attenuation in the depth direction appears at the same time. In this case, a strain distribution correcting function w(r,$\theta$) is developed as the function of "r" and "$\theta$". For example, in the case that the relationship of the formula (14) can be recognized in the depth direction under the measuring condition in embodiment 3, the following formula (20) is to be used as the strain distribution correcting function w(r,$\theta$).

$$w(r,\theta) = w(r) \times w(\theta) = (r/r0) \times (1/\sin\theta) \qquad (20)$$

While the pressure force on both of the side-parts is assumed only as vertical component in the embodiment 4, since pressure component in lateral direction is also generated in reality in this region due to the region pushed away by the movement of the probe 2, it also is possible to set a strain distribution correcting function w(r, $\theta$) considering the lateral component.

Embodiment 5

Since the strain distribution correcting function in the embodiments 1 ~ 4 is the function in compliance with only the coordinate within the FOV range, it is preferable, for example, to obtain the calculated value (configuration value) of the strain distribution correcting function in advance for the coordinate positions of each frame in accordance with the pressed condition of the pressing means and to store them in a memory in the strain distribution correcting function creating unit 18. In this manner, it is possible to perform correction in real time referring to the configuration value corresponding to the coordinate of the calculated strain value.

Also, as for the strain distribution correcting function, appropriate function such as logarithmic function or exponential function can be applied by analyzing pressure measuring condition, without limiting to the function of (1/r) and (1/r$^2$) illustrated in the embodiments 1 ~ 4.

Further, depending on the pressure measuring condition such as the shape of the probe 2 or a pressing target, there are cases that it is difficult to develop an appropriate strain distribution correcting function by modeling due to complexity in transmission of stress distribution. In this case, for example, the strain distribution correcting function creating unit 18 can develop a strain distribution correcting function by a simulation such as the finite element method.

By creating a strain distribution correcting function from an actual measurement value using a phantom and storing the obtained strain distribution correcting function in a memory of the strain distribution correcting function creating unit 18, the strain distribution correcting unit 14 can correct the strain distribution in accordance with the stored strain correcting function.

Embodiment 6

Here, an embodiment for controlling the strain distribution correcting function creating unit 18 via the apparatus control interface 19 in Fig. 1 will be described.

As shown in embodiments 1 ~ 5, it is necessary to switch the strain distribution correcting functions to apply in accordance with the pressure measuring condition such as the kind of pressing means as the probe 2 or a balloon, the shape of FOV range or diffraction angle $\psi$. Given this factor, strain distribution correcting function switching means and ON/OFF switching means are provided to the apparatus control interface unit 19 so that an examiner can switch the functions in accordance with the pressure measuring condition. In concrete terms, the strain distribution correcting function creating unit 18 sets a strain distribution correcting function in accordance with the kind of the probe 2 or pressing means, and stores them in the memory.

For example, in the case that a liner-type probe 21 is provided to an ultrasonic diagnostic apparatus and the measurement mode is switched to linear scanning in the apparatus control interface unit 19, the strain distribution correcting function creating unit 18 applies the strain distribution correcting function of the embodiment 1. Also, in the case that a convex-type transrectal probe 31 is provided to an ultrasonic diagnostic apparatus and the measurement mode is switched to convex scanning in the apparatus control interface unit 19, the strain distribution correcting function creating unit 18 applies the strain distribution correcting function in the embodiment 2. In this manner, by preparing a specific strain distribution correcting function for each configuration of the probe 2 and switching the probe 2, the strain distribution correcting functions can be automatically switched.

Also, when a balloon is used, the region without reflected echo signals up to the film surface can be observed on a B-mode image. By detecting an individual layer region without echoes on a B-mode image, the strain distribution correcting function can be automatically switched to the one for using a balloon.

Also, switching means to determine whether to perform strain distribution correcting process or not can be provided. Further, it can be set to read out the strain information stored in the cine memory unit, switch the strain distribution correcting functions indicated in the respective embodiments, and create the corrected strain information for comparison.

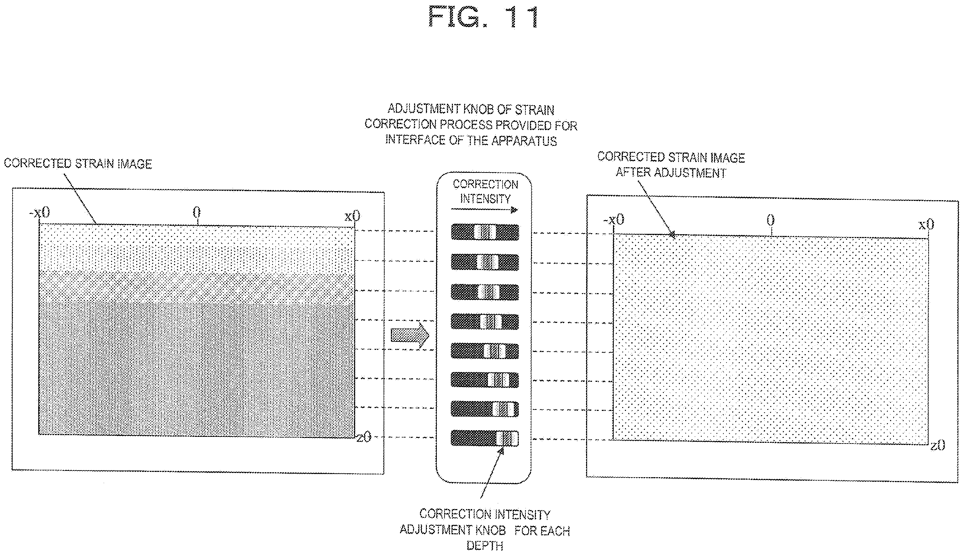

Also, to the common ultrasonic diagnostic apparatus, a function such as TCG (time gain control) or STC (sensitivity time control) is provided for adjusting sensitivity of the received signals in accordance with the measurement depth. These functions are set so that the sensitivity of each position in the measurement depth can be adjusted by the fine adjustment knob. Given this factor, for enabling to individual adjustment for strain distribution correcting functions in accordance with the depth, a fine-adjustment knob (including lateral direction) of strain distribution correcting functions can be provided as shown in Fig. 11. Then, for example, when it is determined that the intensity of correction is small in a deep region, the operation can be carried out to make the correction more effective.

In this case, the fine-adjustment knob for a strain distribution correcting function can be variably adjusted and set on only fine weight from the present selected strain distribution correcting function, and also can be restricted not to make extreme variation.

Also, an adjustment knob of TGC for B-mode images can be switched and replaced as an adjustment knob for strain distribution correcting functions.

Further, not only the correction in the depth direction of w(r), but also adjustment in the angle direction of w($\theta$) (lateral direction) can be made in the same manner.

While an example for correcting strain distribution by setting strain distribution correcting functions is explained in the above respective embodiments, the present disclosure does not have to be limited to the embodiments thereof, corrected strain distribution can be obtained by setting a correcting function of displacement in advance and correcting displacement distribution to use for strain calculation, which can achieve the same effectiveness as the above-described embodiments. In concrete terms, displacement distribution is measured for each pressure measuring condition and a displacement distribution correcting function to make displacement distribution wherein the stress on the contact surface does not get attenuated even in an arbitrary depth is set in advance in a displacement distribution correcting function creating unit (not shown in the diagram). Then a displacement correcting unit (not shown in the diagram) obtains appropriate displacement information regardless of the depth or direction from the pressing means by correcting the displacement distribution acquired by a displacement calculating unit 12 using the displacement distribution correcting function. Then the strain information calculating unit 13 obtains displacement distribution from the corrected displacement information.

## Claims

1. An ultrasonic diagnostic apparatus comprising:

   an ultrasonic intra-luminal probe (2) for transmitting/receiving ultrasonic waves to/from an object (1) to be examined;
   pressing means for adding pressure to biological tissues of the object (1);
   transmission means (3) for transmitting ultrasonic waves to the biological tissues by the ultrasonic probe (2);
   reception means (5) for receiving the reflected echo signals generated from the object by the ultrasonic probe (2);
   strain information calculating means (13) for obtaining strain distribution of biological tissues based on a pair of frame data acquired at different times received by the reception means (5);
   strain image construction means for constructing a strain image based on the strain distribution obtained by the strain information calculating means (13); and
   display means (10) for displaying the strain image,
   **characterized in** further comprising strain distribution correcting means (14) for correcting the strain distribution by a strain distribution correcting function being set in accordance with the pressing condition by the pressing means, wherein
   the pressing means is a balloon which is attached to the ultrasonic probe (2), and which is expanded or contracted by charging water thereto or discharging water therefrom, respectively;
   the balloon is a spherical-shaped balloon (33) or a balloon (41) having a cylindrical shaped membrane; and

if the balloon is the spherical-shaped balloon (33), the strain distribution correcting function is defined as $(r/rO)^2$, and if the balloon is the balloon (41) having the cylindrical-shaped membrane, the strain distribution correction function is defined as $(r/rO)$, where rO is a curvature radius of the balloon in its initial state and r is a radial coordinate of a measurement point in the object (1).

2. The ultrasonic diagnostic apparatus according to claim 1, **characterized by** comprising storing means (20) for acquiring the strain distribution correcting function in accordance with the pressing condition by the pressing means, wherein the strain distribution correcting means (14) is adapted to correct the strain distribution by the stored strain distribution correcting function.

3. The ultrasonic diagnostic apparatus according to claim 1, **characterized by** comprising storing means (20) for acquiring the strain distribution correcting function for each coordinate position of the strain distribution, wherein the strain distribution correcting means (14) is adapted to correct the strain distribution by the stored strain distribution correcting function.

4. The ultrasonic diagnostic apparatus according to claim 1,
wherein the strain distribution correcting function is adapted to correct the strain distribution so that the stress does not get attenuated in an arbitrary depth, based on the stress attenuation amount that works on the biological tissues of the object (1).

5. The ultrasonic diagnostic apparatus according to claim 4,
wherein the strain distribution correcting function is the inverse number of the stress attenuation amount.

6. The ultrasonic diagnostic apparatus according to claim 5,
wherein the strain distribution correcting means is adapted to correct the strain distribution by multiplying the strain distribution by the inverse number of the stress attenuation amount.

7. The ultrasonic diagnostic apparatus according to claim 1,
wherein the strain distribution correcting function is created by calculating stress attenuation amount which attenuates based on the pressing condition of the press means (21; 31).

8. The ultrasonic diagnostic apparatus according to claim 1,
wherein the strain distribution correcting function is created in accordance with the kind of ultrasonic probe (2).

9. The ultrasonic diagnostic apparatus according to claim 1,
wherein the strain distribution correcting function is created based on the shape of a contact surface of the pressing means or the ultrasonic probe (2).

10. The ultrasonic diagnostic apparatus according to claim 1,
wherein the strain distribution correcting function is created based on the distance of the pressing means or the ultrasonic probe (2) from the contact surface.

11. The ultrasonic diagnostic apparatus according to claim 1,
wherein the strain distribution correcting function is created based on a diffraction angle of the elastic waves.

12. The ultrasonic diagnostic apparatus according to claim 1,
wherein the strain distribution correcting function is created based on the radius of the contact surface of the pressing means or the ultrasonic probe (2).

13. The ultrasonic diagnostic apparatus according to claim 1,
wherein the strain distribution correcting function is created based on the size of the pressing means or the ultrasonic probe (2).

14. The ultrasonic diagnostic apparatus according to claim 1, **characterized in** further comprising adjustment means for independently adjusting the strain distribution correcting function for each depth in the object (1).

15. The ultrasonic diagnostic apparatus according to claim 1, **characterized in** further comprising displacement calculating means for correcting displacement distribution of biological tissues acquired based on a pair of frame data by the displacement distribution correcting function set in accordance with the pressing condition by the pressing means, wherein the strain information calculating means (13) obtains the strain distribution based on the corrected displacement distribution.

**Patentansprüche**

1. Ultraschalldiagnosevorrichtung, die Folgendes umfasst:

   eine intraluminale Ultraschallsonde (2) zum Übertragen/Empfangen von Ultraschallwellen zu/von einem zu untersuchenden Objekt (1);
   Druckmittel zum Aufbringen von Druck auf biologische Gewebe des Objekts (1);
   Übertragungsmittel (3) zum Übertragen von Ultraschallwellen auf die biologischen Gewebe durch die Ultraschallsonde (2);
   Empfangsmittel (5) zum Empfangen der reflektierten Echosignale, die durch die Ultraschallsonde (2) von dem Objekt erzeugt werden;
   Mittel (13) zum Berechnen von Belastungsinformationen zum Ermitteln der Belastungsverteilung der biologischen Gewebe auf der Basis eines Paars von Rahmendaten, die zu verschiedenen Zeiten erfasst werden, das durch das Empfangsmittel (5) empfangen wird;
   Mittel zum Konstruieren eines Belastungsbilds zum Konstruieren eines Belastungsbilds auf der Basis der Belastungsverteilung, die durch das Mittel (13) zum Berechnen der Belastungsinformationen ermittelt wird; und
   Anzeigemittel (10) zum Anzeigen des Belastungsbilds,
   **dadurch gekennzeichnet, dass** sie ferner Mittel (14) zum Korrigieren der Belastungsverteilung zum Korrigieren der Belastungsverteilung durch eine Belastungsverteilungskorrekturfunktion, die in Übereinstimmung mit dem Druckzustand durch das Druckmittel eingestellt wird, umfasst, wobei
   das Druckmittel ein Ballon ist, der an der Ultraschallsonde (2) befestigt ist und der jeweils durch Einfüllen von Wasser oder durch Ablassen von Wasser daraus ausgedehnt oder zusammengezogen wird;
   der Ballon ein kugelförmiger Ballon (33) oder ein Ballon (41) ist, der eine zylindrisch geformte Membran aufweist; und
   dann, wenn der Ballon der kugelförmige Ballon (33) ist, die Belastungsverteilungskorrekturfunktion definiert ist als $(r/r0)^2$ und dann, wenn der Ballon der Ballon (41) ist, der die zylindrisch geformte Membran aufweist, die Belastungsverteilungskorrekturfunktion definiert ist als $(r/rO)$, wobei r0 ein Krümmungsradius des Ballons in seinem Anfangszustand ist und r eine radiale Koordinate eines Messpunkts im Objekt (1) ist.

2. Ultraschalldiagnosevorrichtung nach Anspruch 1, **gekennzeichnet durch** das Umfassen von Speichermitteln (20) zum Erfassen der Belastungsverteilungskorrekturfunktion in Übereinstimmung mit dem Druckzustand **durch** das Druckmittel, wobei das Mittel (14) zur Korrektur der Belastungsverteilung ausgelegt ist, die Belastungsverteilung **durch** die gespeicherte Belastungsverteilungskorrekturfunktion zu korrigieren.

3. Ultraschalldiagnosevorrichtung nach Anspruch 1, **gekennzeichnet durch** Speichermittel (20) zum Erfassen der Belastungsverteilungskorrekturfunktion für jede Koordinatenposition der Belastungsverteilung, wobei das Mittel (14) zur Korrektur der Belastungsverteilung ausgelegt ist, die Belastungsverteilung **durch** die gespeicherte Belastungsverteilungskorrekturfunktion zu korrigieren.

4. Ultraschalldiagnosevorrichtung nach Anspruch 1,
   wobei die Belastungsverteilungskorrekturfunktion ausgelegt ist, die Belastungsverteilung auf der Basis des Belastungsdämpfungsgrads, der auf die biologischen Gewebe des Objekts (1) wirkt, derart zu korrigieren, dass die Belastung in einer beliebigen Tiefe nicht gedämpft wird.

5. Ultraschalldiagnosevorrichtung nach Anspruch 4,
   wobei die Belastungsverteilungskorrekturfunktion der inverse Betrag des Belastungsdämpfungsgrads ist.

6. Ultraschalldiagnosevorrichtung nach Anspruch 5,
   wobei das Mittel zur Korrektur der Belastungsverteilung ausgelegt ist, die Belastungsverteilung durch Multiplizieren der Belastungsverteilung mit dem inversen Betrag des Belastungsdämpfungsgrads zu korrigieren.

7. Ultraschalldiagnosevorrichtung nach Anspruch 1,
   wobei die Belastungsverteilungskorrekturfunktion durch Berechnen des Belastungsdämpfungsgrads, der aufgrund des Druckzustands des Druckmittels (21, 31) dämpft, erzeugt wird.

8. Ultraschalldiagnosevorrichtung nach Anspruch 1, wobei die Belastungsverteilungskorrekturfunktion in Übereinstimmung mit dem Typ der Ultraschallsonde (2) erzeugt wird.

9. Ultraschalldiagnosevorrichtung nach Anspruch 1, wobei die Belastungsverteilungskorrekturfunktion auf der Basis der Form einer Kontaktfläche des Druckmittels oder der Ultraschallsonde (2) erzeugt wird.

10. Ultraschalldiagnosevorrichtung nach Anspruch 1, wobei die Belastungsverteilungskorrekturfunktion auf der Basis des Abstands des Druckmittels oder der Ultraschallsonde (2) von der Kontaktfläche erzeugt wird.

11. Ultraschalldiagnosevorrichtung nach Anspruch 1, wobei die Belastungsverteilungskorrekturfunktion auf der Basis eines Beugungswinkels der elastischen Wellen erzeugt wird.

12. Ultraschalldiagnosevorrichtung nach Anspruch 1, wobei die Belastungsverteilungskorrekturfunktion auf der Basis des Radius der Kontaktfläche des Druckmittels oder der Ultraschallsonde (2) erzeugt wird.

13. Ultraschalldiagnosevorrichtung nach Anspruch 1, wobei die Belastungsverteilungskorrekturfunktion auf der Basis der Größe des Druckmittels oder der Ultraschallsonde (2) erzeugt wird.

14. Ultraschalldiagnosevorrichtung nach Anspruch 1, **dadurch gekennzeichnet, dass** sie ferner Einstellungsmittel zum unabhängigen Einstellen der Belastungsverteilungskorrekturfunktion für jede Tiefe im Objekt (1) umfasst.

15. Ultraschalldiagnosevorrichtung nach Anspruch 1, **dadurch gekennzeichnet, dass** sie ferner Mittel zum Berechnen einer Verschiebung zum Korrigieren der Verschiebungsverteilung der biologischen Gewebe, die auf der Basis eines Paars von Rahmendaten erfasst wird, durch die Verschiebungsverteilungskorrekturfunktion, die in Übereinstimmung mit dem Druckzustand des Druckmittels eingestellt wird, umfasst, wobei das Mittel (13) zum Berechnen der Belastungsinformationen die Belastungsverteilung auf der Basis der korrigierten Verschiebungsverteilung ermittelt.

**Revendications**

1. Appareil de diagnostic à ultrasons comprenant :

une sonde intra-luminale à ultrasons (2) pour émettre/recevoir des ondes ultrasonores vers/depuis un objet (1) à examiner ;
un moyen de pressage pour appliquer une pression à des tissus biologiques de l'objet (1) ;
un moyen d'émission (3) pour émettre des ondes ultrasonores vers les tissus biologiques par la sonde à ultrasons (2) ;
un moyen de réception (5) pour recevoir les signaux d'écho réfléchis générés depuis l'objet par la sonde à ultrasons (2) ;
un moyen de calcul d'information de contrainte (13) pour obtenir une distribution de contrainte des tissus biologiques sur la base d'une paire de données de trames acquises à des moments différents et reçues par le moyen de réception (5) ;
un moyen de construction d'image de contrainte pour construire une image de contrainte sur la base de la distribution de contrainte obtenue par le moyen de calcul d'information de contrainte (13) ; et
un moyen d'affichage (10) pour afficher l'image de contrainte,
**caractérisé en ce qu'**il comprend en outre un moyen de correction de distribution de contrainte (14) pour corriger la distribution de contrainte par une fonction de correction de distribution de contrainte qui est fixée en accord avec la condition de pressage par le moyen de pressage, dans lequel
le moyen de pressage est un ballon qui est attaché à la sonde à ultrasons (2) et qui est mis en expansion ou contracté en chargeant de l'eau vers celui-ci eau en déchargeant de l'eau hors de celui-ci, respectivement ; le ballon est un ballon de forme sphérique (33) ou un ballon (41) ayant une membrane de forme cylindrique ; et si le ballon est un ballon de forme sphérique (33), la fonction de correction de distribution de contrainte est définie comme $(r/rO)^2$, et si le ballon est un ballon (41) ayant une membrane de forme cylindrique, la fonction de correction de distribution de contrainte est définie comme $(r/rO)$, dans laquelle rO est un rayon de courbure du ballon dans son état initial, et r est une coordonnée radiale d'un point de mesure dans l'objet (1).

2. Appareil de diagnostic à ultrasons selon la revendication 1,
**caractérisé en ce qu'**il comprend un moyen de stockage (20) pour acquérir la fonction de correction de distribution de contrainte en accord avec la condition de pressage par le moyen de pressage, dans lequel le moyen de correction de distribution de contrainte (14) est adapté à corriger la distribution de contrainte par la fonction de correction de distribution de contrainte stockée.

3. Appareil de diagnostic à ultrasons selon la revendication 1,
**caractérisé en ce qu'**il comprend un moyen de stockage (20) pour acquérir la fonction de correction de distribution de contrainte pour chaque position de coordonnées de la distribution de contrainte, dans lequel le moyen de correction de distribution de contrainte (14) est adapté à corriger la distribution de contrainte par la fonction de correction de distribution de contrainte stockée.

4. Appareil de diagnostic à ultrasons selon la revendication 1,
dans lequel la fonction de correction de distribution de contrainte est adaptée à corriger la distribution de contrainte de telle façon que les tensions ne deviennent pas atténuées à une profondeur arbitraire, basée sur la quantité d'atténuation de tension qui agit sur les tissus biologiques de l'objet (1).

5. Appareil de diagnostic à ultrasons selon la revendication 4,
dans lequel la fonction de correction de distribution de contrainte est l'inverse de la quantité d'atténuation de tension.

6. Appareil de diagnostic à ultrasons selon la revendication 5,
dans lequel le moyen de correction de distribution de contrainte est adapté à corriger la distribution de contrainte en multipliant la distribution de contrainte par l'inverse de la quantité d'atténuation de tension.

7. Appareil de diagnostic à ultrasons selon la revendication 1,
dans lequel la fonction de correction de distribution de contrainte est créée en calculant la quantité d'atténuation de tension qui s'atténue sur la base de la condition de pressage du moyen de pressage (21 ; 31).

8. Appareil de diagnostic à ultrasons selon la revendication 1,
dans lequel la fonction de correction de distribution de contrainte est créée en accord avec le type de sonde à ultrasons (2).

9. Appareil de diagnostic à ultrasons selon la revendication 1,
dans lequel la fonction de correction de distribution de contrainte est créée sur la base de la forme d'une surface de contact du moyen de pressage ou de la sonde à ultrasons (2).

10. Appareil de diagnostic à ultrasons selon la revendication 1,
dans lequel la fonction de correction de distribution de contrainte est créée sur la base de la distance du moyen de pressage ou de la sonde à ultrasons (2) depuis la surface de contact.

11. Appareil de diagnostic à ultrasons selon la revendication 1,
dans lequel la fonction de correction de distribution de contrainte est créée sur la base d'un angle de diffraction des ondes élastiques.

12. Appareil de diagnostic à ultrasons selon la revendication 1,
dans lequel la fonction de correction de distribution de contrainte est créée sur la base du rayon de la surface de contact du moyen de pressage ou de la sonde à ultrasons (2).

13. Appareil de diagnostic à ultrasons selon la revendication 1,
dans lequel la fonction de correction de distribution de contrainte est créée sur la base de la taille du moyen de pressage ou de la sonde à ultrasons (2).

**14.** Appareil de diagnostic à ultrasons selon la revendication 1,
**caractérisé en ce qu'**il comprend en outre un moyen d'ajustement pour ajuster indépendamment la fonction de correction de distribution de contrainte pour chaque profondeur dans l'objet (1).

**15.** Appareil de diagnostic à ultrasons selon la revendication 1,
**caractérisé en ce qu'**il comprend en outre un moyen de calcul de déplacement pour corriger la distribution de déplacement des tissus biologiques acquise sur une paire de données de trames par la fonction de correction de distribution de déplacement fixée en accord avec la condition de pressage par le moyen de pressage,
dans lequel le moyen de calcul d'information de contrainte (13) obtient la distribution de contrainte sur la base de la distribution de déplacement corrigée.

# FIG. 1

EP 2 060 233 B1

EP 2 060 233 B1

# FIG. 2

22

21

STRAIN IN THE
RANGE OF
5 ~ 20%

STRAIN
VARIATION IN
THE RANGE OF
0.2 ~ 1%

PRESSING TARGET

PRESSING TARGET

PRESSING TARGET

(A)

(B)

(C)

FIG. 3

EP 2 060 233 B1

FIG. 4

(A)

(B)

STRAIN
DISTRIBUTION

STRAIN IMAGE

FIG. 5

EP 2 060 233 B1

(A)

(B)

STRAIN
DISTRIBUTION

STRAIN IMAGE

# FIG. 6

(A)

(B)

(C)

EP 2 060 233 B1

FIG. 7

(A)

(B)

EP 2 060 233 B1

FIG. 8

25

FIG. 9

(A)

(B)

FIG. 10

EP 2 060 233 B1

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1629777 A1
- JP P2004229459 B
- WO 2006041050 A1
- JP H5317313 A

**Non-patent literature cited in the description**

- **KARSTEN MARK HILTAWSKY et al.** Freehand ultrasound elastography of breast lesions: Clinical results. *Ultrasound in Med. & Biol.,* 2001, vol. 27 (11), 1461-1469